Europäisches Patentamt

European Patent Office

Office européen des brevets

(19)

(11) Veröffentlichungsnummer : **0 585 201 A1**

(12) **EUROPÄISCHE PATENTANMELDUNG**

(21) Anmeldenummer : **93810561.6**

(22) Anmeldetag : **09.08.93**

(51) Int. Cl.$^5$ : **G01N 33/68,** G01N 33/564

(30) Priorität : **24.08.92 CH 2616/92**

(43) Veröffentlichungstag der Anmeldung : **02.03.94 Patentblatt 94/09**

(84) Benannte Vertragsstaaten : **AT BE CH DE DK ES FR GB GR IE IT LI NL PT SE**

(71) Anmelder : **BMA BIOMEDICALS AG Rheinstrasse 28-32 CH-4302 Augst (CH)**

(72) Erfinder : **Burmeister, Gerd, Dr. Neumattstrasse 97 CH-4455 Zunzgen (CH)**

(54) **Diagnostische Testpackung zur Bestimmung von Proteinen.**

(57)    Die Erfindung betrifft eine diagnostische Testpackung zur Bestimmung und Unterscheidung von akuten und chronischen entzündlichen Zuständen mittels einer Zusammenstellung von spezifisch ausgewählten, standardisierten, rekombinanten Antigenen, spezifisch ausgewählten, selektiv reagierenden, monoklonalen Bindungsantikörpern und spezifisch ausgewählten, selektiv reagierenden, derivatisierten, selektiv reagierenden Nachweisantikörpern.

EP 0 585 201 A1

## GEBIET DER ERFINDUNG

Die Erfindung betrifft eine diagnostische Testpackung zur Bestimmung und Unterscheidung von akuten und chronischen entzündlichen Zuständen, konventionelle Verfahren zur Herstellung dieser Testpackung, die Verwendung der Testpackung und eine diagnostische Methode zur Unterscheidung von akuten chronischen entzündlichen Zuständen in Körperflüssigkeiten.

## HINTERGRUND DER ERFINDUNG

Unter einer Entzündung wird in der Medizin die Reaktion, üblicherweise die Abwehrreaktion, des Organismus und seiner Gewebe gegen verschiedenartige, meistens schädigende Reize verstanden. Als Ziel der Entzündung wird die Beseitigung des schädigenden Reagenses und seiner Folgen angesehen. Gemäss ihrem zeitlichen Verlauf kann man akute, d.h. kurzzeitig auftretende und von vollständiger Wiederherstellung des Organismus gefolgte, von chronischen, d. h. permanent oder zumindest über einen sehr langen Zeitraum vorhandene, gegebenenfalls von zeitlich limitierten, akuten Schüben begleitete, Entzündungen unterscheiden.

Das Entzündungsgeschehen im tierischen und menschlichen Körper äussert sich durch Rötung, Wärme, Schwellung, Schmerzen und andere gestörte Funktionen. Es wird von einer Reihe von Markerpolypeptiden begleitet, die beim Auftreten einer Entzündung von verschiedenen Zellen, insbesondere den dann vermehrt auftretenden Granulozyten, unterschiedlichen Reifegrades exprimiert werden.

So wurde 1980 der Calcium bindende Proteinkomplex L-1 von der Gruppe um Fagerhol als Messparameter für den "Turnover" von Granulozyten beschrieben (1). Es wurde festgestellt, dass das L-1 Protein ca. 5% des gesamten Granulozytenproteins ausmacht, dass es ins Blut abgegeben wird, wo es im Plasma und Serum nachgewiesen werden kann (1,2). Durch zytologische und histologische Untersuchungen wurde es auch in Monozyten (3), in Epithelzellen und im Gefässendothelium nachgewiesen (4,22).

Für das L-1 Protein wurden unterschiedliche Molekulargewichte beschrieben. In den ersten Arbeiten (1,2) wurden Molekulargewichte von 51 kD und später (5) von 36.5 kD angegeben. Von Letzterem wurde vermutet, es sei aus drei Untereinheiten von je 12.5 kD zusammengesetzt.

In der Arbeitsgruppe um Sorg (6) wurden die als MRP 8 und MRP 14 (MRP = MIF Related Protein, MIF = Macrophage Migration Inhibiting Factor) bezeichneten Proteine vom ungefähren Molekulargewicht 8 000 bzw. 14 000 entdeckt. Diese beiden Proteine konnten von Odink et al. (7) und Lagasse et al. (8, 9) kloniert werden. Später wurde erkannt, dass es sich um Untereinheiten des L-1 Proteins handelt.

In Abhängigkeit vom Calciumgehalt einer Lösung können diese beiden Untereinheiten die dimeren, homogenen Komplexe MRP 8 - MRP 8 und MRP 14 - MRP 14, den dimeren, heterogenen Komplex MRP 8 - MRP 14, sowie polymere homogene und heterogene Komplexe bilden, wodurch die gefundenen unterschiedlichen Molekulargewichte für die L-1 Proteine eine bessere Erklärung fanden (10). Das L-1 Protein vom Molekulargewicht 36.5 kD setzt sich demnach zusammen aus den Molekulargewichten von zwei Molekülen MRP 8 (MW 10295) und einem Molekül MRP 14 (MW 13049). Die klonierten Moleküle liessen erkennen, dass MRP 8 und MRP 14 zu den Calcium bindenden Proteinen der S100 Proteinfamilie gehören (9). Aufgrund partieller Sequenzähnlichkeiten wurde MRP 8 mit dem Cystic Fibrosis Antigen und MRP 14 mit NIF-1 und NIF-2 (Neutrophil-Immobilising Factor) in Verbindung gebracht (11, 12, 21). Weitere homologe Bezeichnungen sind p8,14 (22).

Seit 1980 deckten eine Reihe von Publikationen die klinische Relevanz des Vorkommens der L-1 Proteine im Plasma und Serum und bei zytologischen und histologischen Untersuchungen auf, wobei die L-1 Konzentrationen mit den Konzentrationen an C-reaktivem Protein (CRP) und der Erythrozytensedimentationsrate (ESR) im Blut in Beziehung gesetzt wurden (1, 2, 3, 13, 14, 15, 16, 19). Die gefundenen Korrelationskoeffizienten von etwa 0.4 bis 0.7 waren aber eher niedrig. In manchen Studien war jedoch eine gewisse Signifikanz zwischen den L-1 Proteinen, CRP und ESR Messungen festgestellt worden (z. B. 14). In Körperflüssigkeiten können erhöhte Mengen an L-1 Proteinen bei bakteriellen aber nicht bei viralen Infektionen gemessen werden (13).

Studien mit monospezifisch, polyklonalen Antikörpern, die zeigen sollten, ob der Gehalt an MRP 8 oder MRP 14 mit bestimmten Krankheiten korreliert werden könnte, wurden bei verschiedenen Erkrankungen durchgeführt (7, 16, 17, 20). So konnte gezeigt werden, dass MRP 8 und MRP 14 bei gewissen Erkrankungen in unterschiedlichen Mengen im Gewebe exprimiert werden (18) und, dass im zeitlichen Verlauf einer normal verlaufenden akuten Entzündung, eine Korrelation zwischen dem Auftreten dieser Proteine und der Phase der Entzündung besteht (17). Bei chronischen Entzündungen, wie der rheumatoiden Arthritis, traten Zellen auf, die entweder MRP 8 oder MRP 14 enthielten, jedoch wurden keine Zellen nachgewiesen, die Heterokomplexe aus MRP 8 und MRP 14 enthielten (7, 16). Heterokomplexe enthaltende Zellen sind mit dem monoklonalen Antikörper 27 E 10 nachweisbar (23).

Wegen der undifferenzierten Bindungsfähigkeit der zwar monospezifischen, aber polyklonalen Antikörper, die ja sowohl auf alle Epitope der singulären MRP 8 und MRP 14 Proteine, als auch auf diejenigen ihrer homologen und heterologen Komplexe ansprechen, konnte zwischen dem Gehalt an den genannten Untereinheiten und ihren verschiedenen Komplexen (z. B. dem L-1 Protein) im Plasma oder Serum nicht unterschieden werden. Diese Tatsache trifft auch auf die Messungen zu, in denen polyklonale Antikörper gegen das L-1 Protein Verwendung fanden (z. B. 1, 19).

Nach Herstellung der rekombinanten, reinen Peptide MRP 8 und MRP 14 gelang es, auch spezifische, auf eine Reihe verschiedener Epitope gerichtete, monoklonale Antikörper herzustellen, die entweder spezifisch nur die Einzelkomponenten oder gegebenenfalls, wenn die entsprechenden Epitope frei lagen, auch die aus MRP 8 und MRP 14 zusammengesetzten Komplexe erkannten. Es war ein glücklicher Zufall, dass der von der Arbeitsgruppe um Sorg entdeckte monoklonale Antikörper 27 E 10 ausschliesslich heterologe Komplexe aus MRP 8 und MRP 14 erkannte (23).

Es war bisher nicht möglich, die bekannten natürlichen oder rekombinanten Peptide MRP 8, MRP 14 und die daraus zusammengesetzen Komplexe, incl. L-1, sowie die damit reagierenden entsprechenden polyklonalen oder monoklonalen Antikörper in eindeutiger Weise für diagnostische Zwecke einzusetzen. Insbesondere konnte keine Unterscheidung zwischen akuten und chronischen entzündlichen Zuständen getroffen werden. Auf medizinischer Seite besteht ein Bedarf, diese beiden Zustände zu unterscheiden, um sowohl eine Prognose über den zukünftigen Krankheitsverlauf stellen zu können, als auch die Behandlungsweise danach auszuwählen und den Erfolg der Therapie zu überprüfen.

## AUFGABE DER ERFINDUNG

Der vorliegenden Erfindung liegt die Aufgabe zu Grunde, eine diagnostische Testpackung und eine diagnostische Methode zur Verfügung zu stellen, die es erlauben, akute von chronischen entzündlichen Zuständen zu unterscheiden. Diese Aufgabe kann überraschenderweise unter Zuhilfenahme der rekombinanten Proteine MRP 8 und MRP 14 und ausgewählten, geeigneten monoklonalen Antikörpern, die in der vorliegenden Testpackung enthalten sind, in befriedigender Weise gelöst werden.

## DETAILLIERTE BESCHREIBUNG DER ERFINDUNG

Die Erfindung betrifft eine diagnostische Testpackung zur Bestimmung und Unterscheidung von akuten und chronischen entzündlichen Zuständen mittels einer Zusammenstellung von spezifisch ausgewählten, standardisierten, rekombinanten Antigenen, spezifisch ausgewählten, selektiv reagierenden, monoklonalen Bindungsantikörpern und spezifisch ausgewählten, selektiv reagierenden, derivatisierten Nachweisantikörpern.

Aus einer Reihe von monoklonalen Antikörpern gegen die Antigene MRP 8 und MRP 14 wurden solche ausgewählt, die in einem immunologischen Test entweder nur das Monomer oder Homopolymer von MRP 8 oder das Monomer oder Homopolymer von MRP 14, jedoch nicht die Heterokomplexe aus MRP 8 und MRP 14 erkennen. Weiterhin wurde ein monoklonaler Antikörper ausgewählt, der ein Epitop erkennt, das sich nur im Heterokomplex MRP 8 - MRP 14 (L-1) befindet. Diese monoklonalen Antikörper sind Mab 8-5C2, Mab S 36.48 und Mab 27 E 10. Sie werden als Bindungsantikörper bezeichnet.

Weiterhin wurden drei monoklonale Antikörper ausgewählt, die die drei Antigen/Antikörper Komplexe erkennen. Diese, als Nachweisantkörper bezeichneten Antikörper, werden derivatisiert, z. B. mit Biotin, damit sie ihrerseits und damit die Antigen/Antikörperkomplexe, nachgewiesen werden können. Es handelt sich hierbei um die, beispielsweise biotinylierten, monoklonalen Antikörper S 13.67, S32.2 und S 36.48.

Es werden demnach die in der **Tabelle 1** zusammengestellten Testkombinationen benutzt:

| Tabelle 1 | | | |
|---|---|---|---|
| rekomb. Antigene | Bindungs-Mab | Nachweis-Mab | erkannte Antigene |
| MRP 8 | 8-5C2 | S 13.67 | MRP 8 Monomer und Homopolymer |
| MRP 14 | S 36.48 | S 32.2 | MRP 14 Monomer und Homopolymer |
| MRP 8/14 | 27 E 10 | S 36.48 | MRP 8/14 Heterodimer und Heteropolymer |

Überraschenderweise lassen sich mit Hilfe dieser Kombinationen, durch Bestimmung der in Körperflüssigkeiten vorhandenen Mengen MRP 8, MRP 14 und Heterokomplex MRP 8 - MRP 14 akute von chronischen Entzündungszuständen unterscheiden. Die Zuordnungen werden anhand der **Tabelle 2** getroffen.

**Tabelle 2**

| Krankheits-zustände | MRP 8 ng/ml | MRP 14 ng/ml | MRP 8/14 ng/ml |
|---|---|---|---|
| 1. Normalfall, keine Entzündg. | 0 | 0 - 50 | 500 - 3000 |
| 2. akute Entzündung | 0 | 70 - 500 | 3000 - 40000 |
| 3. chronische Entzündung mit akutem Schub | 10 - 160 | 70 - 800 | 4000 - 40000 |
| 4. chronische Entzündung ohne akuten Schub | 10 - 160 | 70 - max.300 | 1000 - 3000 |

Die ng/ml Angaben sind ca.-Angaben

**Kurze Beschreibung der Figuren**

FIG. 1 zeigt die Anordnung zweier Verdünnungsreihen eines rekombinanten Standardantigens auf einer Testplatte mit 8 mal 12 Vertiefungen.

FIG. 2 zeigt drei typische Eichkurven von reinen, rekombinanten Antigenen MRP 8, MRP 14 und dem 2:1-Komplex aus MRP 8 und MRP 14, die zur Bestimmung der entsprechenden natürlichen Antigene in Körperflüssigkeiten verwendet werden können.

**Die Testpackungen**

Die Erfindung betrifft insbesondere eine diagnostische Testpackung zur Bestimmung und Unterscheidung von akuten und chronischen entzündlichen Zuständen, enthaltend

a) als Standardantigene, die rekombinanten Peptide MRP 8, MRP 14 und den Komplex aus MRP 8 und MRP 14,

b) als Bindungsantikörper, die monoklonalen Antikörper Mab 8-5C2, Mab S-36.48 und Mab 27E10,

c) als Nachweisantikörper, von Mab S 13.67, Mab S 32.2 und Mab S 36.48 abgeleitete; zum Nachweis geeignet derivatisierte monoklonale Antikörper und gegebenenfalls

d) ein Reagenz zum Sichtbarmachen der Nachweisantikörper.

Die in der Testpackung enthaltenen recombinanten Standardantigene, Bindungsantikörper und derivatisierten Nachweisantikörper sind bekannt oder auf bekannte Weise herstellbar.

Der Komplex aus den beiden Antigenen MRP 8 und MRP 14 bildet sich ohne weitere Manipulationen beim Auflösen von MRP 8 und MRP 14 im Verhältnis 2:1 in einem geeigneten Puffer, z. B. in physiologischem Phosphatpupffer pH 7.2. Er enthält etwa 82 % Heterokomplex MRP 8 - MRP 14, 7 % freies MRP 8 und 11 % freies MRP14, bzw. deren Homopolymere. Die rekombinanten Standardantigene werden in lyophilisierter Form in Vials zur Verfügung gestellt. Zur Bestimmung eines Antigens wird eine Testplatte benötigt.

Die Menge an rekombinanten Standardantigen in einem Vial wird so bemessen, dass nach der Rekonstitution mit Aqua bidest. die Konzentration 1000 ng/ml beträgt. Beispielsweise enthält ein Vial 250 ng rekombinantes MRP 8, 250 ng rekombinantes MRP 14 und 250 ng einer 2:1 Mischung aus rekombinantem MRP 8 - MRP 14. Die Anzahl Vials pro Testpackung richtet sich nach der Anzahl Proben die mit der Testpackung analysiert werden soll. Beispielsweise enthält eine Testpackung 2 oder 10 Vials an rekombinanten Antigenen.

Die Menge an Bindungsantikörper in einem Vial wird, in Abhängigkeit von der Anzahl Proben, die mit der Testpackung analysiert werden soll, so bemessen, dass, nach der Rekonstitution mit Aqua bidest. und entsprechender Verdünnung mit Pufferlösung, die Konzentrationen genügend hoch sind, um eine entsprechende Menge an Antikörpern zu binden. Beispielsweise betragen die Endkonzentrationen an Mab 8-5C2 und Mab 27 E 10 4 ug/ml und an S 36.48 6 ug/ml. In einer Testpackung für zwei, bzw. 10 Platten, sind beispielsweise pro Vial 60, bzw. 220 ug Mab 8-5C2, die gleichen Mengen Mab 27 E 10 und 90, bzw. 330 ug Mab S 36.48 enthalten. Eine Testpackung enthält jeweils ein Vial pro Bindungsantikörper.

Die Nachweisantikörper sind auf geeignete Weise derivatisiert, z. B. in Form eines Flourescenzderivates mit Fluoresceinisothiocyanat (FITC) oder Phycoerythrein (PE), eines Enzymderivates mit Meerrettichperoxidase, Phospathase, Glucosidase oder Galactosidase, oder bevorzugt eines Derivates mit Biotin.

Die Menge an derivatisiertem Nachweisantikörper in einem Vial wird, in Abhängigkeit von der Anzahl Proben die mit der Testpackung analysiert werden soll, so bemessen, dass, nach der Rekonstitution mit Aqua bidest. und entsprechender Verdünnung mit Pufferlösung, die Konzentrationen genügend hoch sind, um an eine entsprechende Menge Antikörper zu binden. Beispielsweise betragen die Endkonzentrationen an biotinylierten Mab S 13.67, Mab S 32.2 und Mab S 36.48 0.5 bis 1 ug/ml. In einer Testpackung für zwei Platten sind beispielsweise pro Vial 15 ug biotinylierte Mab S 13.67, Mab S 32.2 und Mab S 36.48 enthalten. Eine Testpackung enthält jeweils ein Vial pro Bindungsantikörper.

Die Fluoreszenzderivate der Nachweisantiköper liegen nicht in lyophilisierter Form, sondern üblicherweise in entsprechenden Pufferlösungen vor.

Die diagnostischen Testpackungen der vorliegenden Erfindung können zur Bestimmung von beliebig vielen Proben ausgelegt sein. Sie enthalten dementsprechend beispielsweise 1-20, bevorzugt 2 oder 10, Vials mit standardisierten Mengen von rekombinanten Antigenen, aber jeweils nur ein Vial mit einer entsprechenden Menge Antikörper.

Die diagnostische Testpackung kann gegebenenfalls Reagentien (Substrate) zum Nachweis der derivatisierten Antikörper umfassen, z. B. Extravidin-Peroxidase und 2,2'-Azino-bis-(3ethylbenzthiazolin-6-sulfonsäure) zum Nachweis von biotinylierten Antikörpern.

Die diagnostische Testpackung enthält üblicherweise eine Anweisung zur Benutzung.

## Verfahren zur Herstellung der Testpackung

Die Erfindung betrifft ebenfalls ein Verfahren zur Herstellung einer diagnostischen Testpackung gemäss vorstehender Beschreibung, dadurch gekennzeichnet, dass man sie auf konventionelle Weise herstellt.

Die Vials werden mit den Lösungen der rekombinanten Antigene, bzw. Antkörper, z. B. in Pufferlösungen, wie physiologischem Phosphatpuffer pH 7.2, in den vorgesehenen Mengen und Konzentrationen beschickt und auf konventionelle Weise lyophilisiert und luftdicht, gegebenenfalls unter einem Inertgas, wie Stickstoff, verschlossen. Die Lösungen der Fluorescenzderivate der Nachweisantikörper werden nicht lyophilisiert, sondern in flüssiger Form luftdicht, gegebenenfalls unter Stickstoff, verschlossen. Die Vials werden etikettiert und in geeignete Behälter abgefüllt.

## Verwendung

Die Erfindung betrifft ebenfalls die Verwendung einer Kombination

a) der rekombinanten Peptide MRP 8, MRP 14 und des Komplexes aus MRP 8 und MRP 14 als Standardantigene,

b) der monoklonalen Antikörper Mab 8-5C2, Mab S-36.48 und Mab 27E10 als Bindungsantikörper und

c) von Mab S 13.67, Mab S 32.2 und Mab S 36.48 abgeleiteter, zum Nachweis geeignet derivatisierter monoklonale Antikörper als Nachweisantikörper, zur Bestimmung und Unterscheidung von akuten und chronischen entzündlichen Zuständen und zur Herstellung einer diagnostischen Testpackung zur Bestimmung und Unterscheidung von akuten und chronischen entzündlichen Zuständen.

## Diagnostische Methode

Die Erfindung betrifft ebenfalls eine diagnostische Methode zur Bestimmung und Unterscheidung von akuten und chronischen entzündlichen Zuständen in Körperflüssigkeiten, dadurch gekennzeichnet, dass man mit Hilfe der rekombinanten Standardantigene MRP 8, MRP 14 bzw. der Mischung dieser beiden Antigene im Molverhältnis 2:1, den Bindungsantikörpern Mab 8-5C2, Mab S-36.48 bzw. Mab 27E10 und den zum Nachweis geeignet derivatisierten Mab S 13.67, Mab S 32.2 bzw. Mab S 36.48 für die drei Standardantigene Eichkurven erstellt, in einer geeigneten Körperflüssigkeit (Probe) die darin vorhandene Menge von MRP8, MRP 14 und dem Heterokomplex von MRP 8 und MRP 14 mittels der monoklonalen Bindungsantikörper und derivatisierten Nachweisantikörper durch einen immunologischen Test bestimmt, und durch Vergleich mit der Standardkurve und einer Tabelle die Auswertung vornimmt.

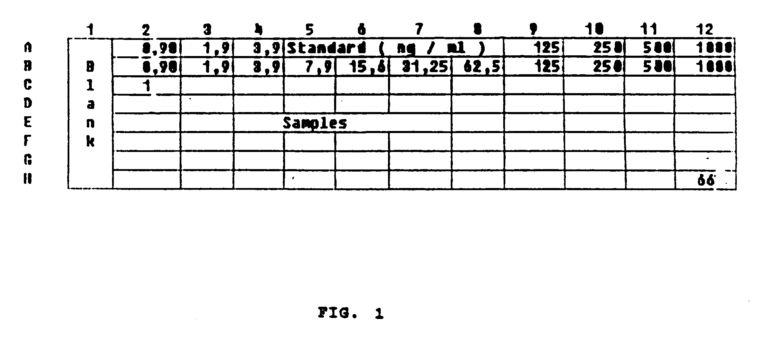

Die immunologischen Tests werden auf üblichen Testplatten durchgeführt, z. B. NUNC MAXISORB Platten, die beispielsweise Anordnungen von 8 mal 12 Vertiefungen (96 Löcher) aufweisen (siehe z. B. **FIG. 1).**

Für jede körpereigene Testflüssigkeit (Probe) muss auf drei Testplatten der Gehalt an natürlichen Antigenen MRP 8, MRP 14 und dem Komplex MRP 8 - MRP 14 bestimmt werden, um zu einer klaren medizinischen Aussage kommen zu können. Die pro Testplatte aufzutragenden Bindungsantikörper, Antigene und Nachweisantikörper, die in dieser Reihenfolge aufgetragen werden, gehen aus der **Tabelle 1** hervor.

Beispielsweise dient Testplatte 1 zur Bestimmung von monomerem und homopolymerem Antigen MRP8. Sie wird nacheinander beschichtet mit dem Bindungsantikörper Mab 8-5C2, dem rekombinantem Antigen MRP 8, den Proben in geeigneter Verdünnung und dem biotinylierten Nachweisantikörper Mab S 13.67.

Testplatte 2 dient beispielsweise zur Bestimmung von monomerem und homopolymerem Antigen MRP 14. Sie wird nacheinander beschichtet mit dem Bindungsantikörper Mab S 36.48, dem rekombinantem Antigen MRP 14, den Proben in geeigneter Verdünnung und dem biotinylierten Nachweisantikörper S 32.2.

Testplatte 3 dient beispielsweise zur Bestimmung von heterodimeren und heteropolymeren Antikörpern MRP 8 - MRP 14. Sie wird nacheinander beschichtet mit dem Bindungsantikörper Mab 27 E 10, der Mischung aus dem Antikörper MRP 8 und MRP 14, den Proben in geeigneter Verdünnung und dem biotinylierten Nachweisantikörper Mab S 36.48.

**Bereitung der Testlösungen**

Die Antikörper werden in den Vials in Aqua bidest. gelöst. Die Lösungen der Bindungsantikörper (a) werden, z. B. mit Carbonatpuffer pH 9.5, auf eine geignete Konzentration weiter verdünnt. Die Lösungen der Nachweisantikörper (b) werden, z. B. mit physiologischem Phosphatpuffer/Kochsalz pH 7.2 enthaltend 0.1 % Tween 20 (FBS-Tween 20), auf eine geeignete Konzentration weiter verdünnt. Die Lösungen der Antigene (c) werden durch Zugabe von Aqua bidest. auf eine geeignete Konzentration eingestellt.

**Auftragen der Bindungsantikörper**

Für jeden Bindungsantikörper wird eine Testplatte benutzt. Die Vertiefungen der Testplatten werden mit einer entsprechenden Menge der Lösung (a) beschickt, eine genügend lange Zeit bei 37° C inkubiert, mit physiologischem Phosphatpuffer PBS-Tween 20 gewaschen, mit der Waschlösung eine genügend lange Zeit, z. B. 1 Stunde, bei 37° C inkubiert und hierauf einmal wie zuvor gewaschen.

**Auftragen der Standardantigene**

Nach dem Auftragen der Bindungsantikörper wird pro Testplatte eines der Standardantigene in einer oder bevorzugt zwei Verdünnungsreihen, z. B. gemäss FIG. 1, aufgetragen. Pro Antigen wird eine Platte benutzt. Beispielsweise wird das rekombinante Antigen MRP 8 auf die Testplatte 1, MRP 14 auf die Testplatte 2 und der Antigenkomplex MRP 8 - MRP 14 auf die Testplatte 3 aufgebracht.

**Auftragen der Proben**

Die Proben der Körperflüssigkeiten, in denen der Gehalt an natürlichen Antigenen MRP 8, MRP 14 und dem Komplex MRP 8-MRP 14 (dieser Komplex entspricht dem L-1 Protein) bestimmt werden soll, werden entweder unverdünnt oder, je nach vermutetem Gehalt an Antigen, etwa gemäss **Tabelle 3,** mit PBS-Tween 20 verdünnt.

| Tabelle 3 | | |
|---|---|---|
| Probe | ungefähre Verdünnung | Bemerkungen |
| EDTA-Plasma | 1/10 bis 1/100 | kein Heparinplasma benutzen |
| Synovialflüssigkeit | 1/100 bis 1/10,000 | |
| Speichel | 1/100 | |
| Exhalat | unverdünnt oder bis zu 1/5 | |

Nach Beschickung mit den Lösungen der Proben, werden die Platten eine genügend lange Zeit bei 37°C inkubiert und mit PBS-Tween 20 gewaschen.

Jede Probe wird auf alle drei Testplatten aufgetragen.

**Auftragen der Nachweisantikörper**

Die Vertiefungen auf allen Platten werden jetzt mit der Lösung der derivatisierten Nachweisantikörper (b) beschickt und die Platten, nach Inkubation bei 37°C, z. B. mit PBS-Tween 20, gewaschen.

**Quantitative Bestimmung der gebildeten Komplexe**

Zur quantitativen Bestimmung der gebildeten Komplexe aus Bindungsantiköper-Antigen-Nachweisantiköper wird ihr Gehalt in den einzelnen Vertiefungen, z. B. fluorimetrisch, gemessen. Diese Messung erfolgt, je nach Derivatisierung der Nachweisantiköper, entweder direkt oder nach entsprechender Behandlung. Beispielsweise kann die Messung im Falle eines Fluoreszenzderivates sofort im Fluorimeter durchgeführt werden. Im Falle eines Enzym-konjugierten Nachweisantikörpers wird zunächst mit einer dem Enzym entsprechenden Substratlösung, wie ABTS (siehe unten) bei mit Peroxidase derivatisiertem Nachweisantikörper, eine genügend lange Zeit, z. B. eine Stunde bei 37° C, inkubiert und hierauf das Produkt aus der enzymatischen Reaktion photometrisch bestimmt. Im Falle eines Biotinderivates folgt eine etwa 30-minütige Inkubation bei 37° C mit einem Avidin-Enzym-Konjugat, z. B. Extravidin-Peroxidase, und nach Waschung, z. B. mit PBS-Tween 20, Inkubation mit dem Substrat ABTS [2,2'-Azino-bis(3-ethylbenzthiazolin-6-sulfonsäure)] und Wasserstoffperoxid und hierauf die photometrische Bestimmung, z. B. durch Messung der Extinktionen bei 405 nm, z. B. mittels eines EIA-Readers.

**Auswertung**

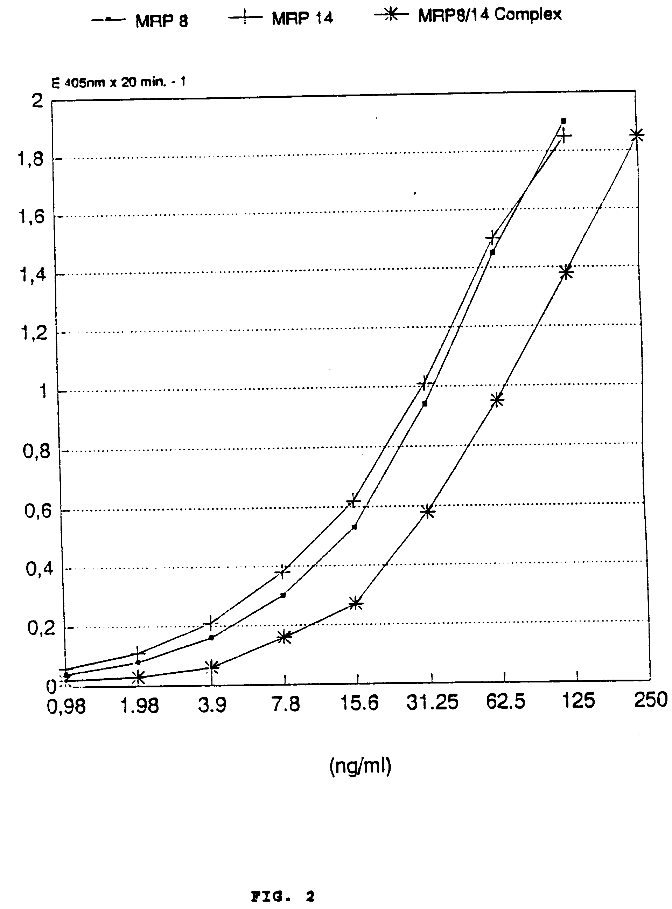

Die Mittelwerte der Extinktionen der beiden Reihen Standardantigene werden gegen die Konzentrationen der Standardantigene in einem Diagramm zu Kurven aufgetragen, die als Eichkurven zur Bestimmung der Konzentrationen der entsprechenden natürlichen Antigene in den Proben dienen. Drei typische Eichkurven sind in **FIG. 2** abgebildet.

Die Konzentrationen der Antigene in den Proben sollten so gewählt werden, dass die sich ergebenden Extinktionen im Bereich des steilen Kurvenanstieges liegen. Der Gehalt der Proben an den natürlichen Antigenen kann dann mit guter Genauigkeit abgelesen, bzw. unter Berücksichtigung der Verdünnungen berechnet werden.

Mittels der **Tabelle 2** wird die Zuordnung getroffen, ob es sich um einen Normalfall, d. h. gesunden Patienten, oder einen Patienten mit akuter Entzündung, chronischer Entzündung mit akutem Schub oder chronischer Entzündung ohne akuten Schub handelt.

Chronische Entzündungen, wie chronische Polyarthritits, sind durch das Auftreten von erhöhten Mengen MRP 8, MRP 14 und Normalwerten von MRP 8 - MRP 14 Heterokomplexen charakterisiert.

Chronische Entzündungen im akuten Schub sind durch das Auftreten erhöhter Mengen MRP 8, MRP 14 und dem Heterokomplex daraus gekennzeichnet.

Bei nicht chronisch verlaufenden, akuten Entzündungen treten nur MRP 14 und der Heterokomplex, jedoch kein MRP 8 auf.

Sollten anormal niedrige Werte des MRP 8 - MRP 14 Komplexes gemessen werden, bei gleichzeitigem Vorliegen einer mit anderen Parametern festgestellten Entzündung, dann kann dieser Zustand (charakterisiert durch eine Linksverschiebung im Blutbild) durch eine ungenügende Anzahl an reifen Granulozyten bedingt sein.

Literatur

1. Fagerhol et al., 1980, Scand. J. Haematol. 24: 393-398
2. Fagerhol et al., 1980, Bull. Eur. Physiopath. 16: 273-281
3. Dale et al., 1985, Am. J. Pathol. 84: 24-34
4. Gabrielsen et al., 1986, J. Am. Acad. Dermatol. 15: 173-179
5. Dale et al., 1983, Eur. J. Biochem. 134: 1 - 6
6. Burmeister et al., 1986, Immunobiology 171:461-474
7. Odink et al., 1987, Nature 330: 80-82
8. Lagasse und Clerc, 1988, Mole. Cellular Biol. 8: 2402-2410
9. Lagasse, 1991, in "Novel Calcum Binding Proteins", pp 225-236, C. W. Heizmann ed., Springer Verlag
10. Teigelkamp et al., 1991, J. Biol. Chem. 266: 13462-13467
11. Anderson, 1988, Nature 332: 688
12. Freemont, et al., Nature 339: 516

13. Sander et al., 1984, Scand. J. Clin. Lab. 44: 357-362

14. Berntzen et al., 1988, Scand. J. Rheumatol. 76: 251-256

15. Berntzen et al., 1989, J. Rheumatol. 16: 1416-1420

16. Zwadlo et al., 1988, Clin. Exp. Immunol. 72: 510-515

17. Mues et al., 1990, Eur. Heart J. 11: 619-627

18. Delabie et al., 1990, Clin. Exp. Immunol. 81:123-126

19. Berntzen et al., 1991, J. Rheumatol. 81: 133-138

20. Brüggen und Cerletti, 1991, "Novel Calcium Binding Proteins", pp 237-247, C. W. Heizmann ed., Springer Verlag

21. Watt et al., 1983, Immunol. 48: 79-86

22. Hogg et al., 1989, Eur. J. Immunol. 19: 1053-1061

23. Bhardwaj et al., 1992, Eur. J. Immunol., in press Die folgenden Beispiele erläutern die Erfindung ohne sie zu beschränken.

**Beispiel 1: Testpackung zur Bestimmung der Antigene in 132 Proben**

Eine diagnostische Testpackung zur Bestimmung der Antigene in 2 mal 66 Proben auf sechs Testplatten mit je 8 mal 12 Vertiefungen unfasst:

2 Vials enthaltend je 250 ng rekombinantes MRP 8,

2 Vials enthaltend je 250 ng rekombinantes MRP 14,

2 Vials enthaltend je 250 ng einer Mischung aus MRP 8 und MRP 14 im Verhältnis 2:1,

1 Vial enthaltend 60 ug Bindungsantikörper Mab 8-5C2,

1 Vial enthaltend 90 ug Bindungsantikörper Mab S 36.48,

1 Vial enthaltend 60 ug Bindungsantikörper Mab 27E10,

1 Vial enthaltend 15 ug biotinylierten Mab S 13.67,

1 Vial enthaltend 15 ug biotinylierten Mab S 32.2, und

1 Vial enthaltend 15 ug biotinylierten Mab S 36.48.

Die Vials werden hergestellt, indem man sie mit einer Lösung der rekombinanten Antigene, bzw. Antikörper, in physiologischem Phosphatpuffer pH 7.2 beschickt und bis zur Trockne lyophilisiert.

Die Testpackung kann zusätzlich das Nachweisreagens Extravidin-Peroxidase umfassen, sowie gegebenenfalls sechs Testplatten, z. B. Nunc Maxisorb Platten. Die Testpackung enthält üblicherweise einen Beipackzettel mit der Beschreibung zur Durchführung der Bestimmungen.

**Beispiel 2: Testpackung zu Bestimmungen der Antigene in 660 Proben**

Eine diagnostische Testpackung zur Bestimmung der Antigene in 660 Proben auf dreissig Testplatten mit je 8 mal 12 Vertiefungen umfasst:

10 Vials enthaltend je 250 ng rekombinantes MRP 8,

10 Vials enthaltend je 250 ng rekombinantes MRP 14,

10 Vials enthaltend je 250 ng einer Mischung aus MRP 8 und MRP 14 im Verhältnis 2:1,

1 Vial enthaltend 220 ug Bindungsantikörper Mab 8-5C2,

1 Vial enthaltend 330 ug Bindungsantikörper Mab S 36.48,

1 Vial enthaltend 220 ug Bindungsantikörper Mab 27E10,

1 Vial enthaltend 100 ug biotinylierten Mab S 13.67,

1 Vial enthaltend 100 ug biotinylierten Mab S 32.2, und

1 Vial enthaltend 50 ug biotinylierten Mab S 36.48.

Die Vials werden hergestellt, indem man sie mit einer Lösung der rekombinanten Antigene, bzw. Antikörper, in physiologischem Phosphatpuffer pH 7.2 beschickt und bis zur Trockne lyophilisiert.

Die Testpackung kann zusätzlich das Nachweisreagens Extravidin-Peroxidase umfassen, sowie gegebenenfalls dreissig Testplatten, z. B. Nunc Maxisorb Platten. Die Testpackung enthält üblicherweise einen Beipackzettel mit der Beschreibung zur Durchführung der Bestimmungen.

**Beispiel 3: Durchführung der Bestimmungen**

Für jede körpereigene Testflüssigkeit (Probe) muss auf drei Testplatten der Gehalt an MRP 8, MRP 14 und dem Komplex MRP 8 - MRP14 bestimmt werden, um zu einer klaren medizinischen Aussage kommen zu können.

Testplatte 1 dient zur Bestimmung von monomerem und homopolymerem Antigen MRP8. Sie wird nacheinander beschichtet mit dem Bindungsantikörper Mab 8-5C2, dem rekombinantem Antigen MRP 8, den Proben in geeigneter Verdünnung und dem biotinylierten Nachweisantikörper Mab S 13.67.

Testplatte 2 dient zur Bestimmung von monomerem und homopolymerem Antigen MRP 14. Sie wird nacheinander beschichtet mit dem Bindungsantikörper Mab S 36.48, dem rekombinantem Antigen MRP 14, den Proben in geeigneter Verdünnung und dem biotinylierten Nachweisantikörper S 32.2.

Testplatte 3 dient zur Bestimmung von heterodimeren und heteropolymeren Antikörpern MRP 8 - MRP 14. Sie wird nacheinander beschichtet mit dem Bindungsantikörper Mab 27 E 10, der Mischung aus dem Antikörper MRP 8 und MRP 14, den Proben in geeigneter Verdünnung und dem biotinylierten Nachweisantikörper Mab S 36.48.

## Bereitung der Testlösungen

Die Antikörper werden durch Zugabe von 0.5 ml Aqua bidest. zu jedem Vial gelöst. Die Lösungen (a) der Bindungsantikörper 8-5C2 und 27 E 10 werden mit Carbonatpuffer pH 9.5 bis auf eine Konzentration von 4 ug/ml und diejenige des Bindungsantikörpers S 36.48 bis auf eine Konzentration von 6 ug/ml weiter verdünnt. Die Lösungen (b) der Nachweisantikörper werden mit physiologischem Phosphat-puffer/Kochsalz pH 7.2 enthaltend 0.1 % Tween 20 (PBS-Tween 20) auf eine Konzentration von 0.5-1 ug/ml weiter verdünnt. Die Lösungen (c) der Antigene werden durch Zugabe von Aqua bidest. auf eine Konzentration von 1000 ng/ml eingestellt.

## Auftragen der Bindungsantikörper

Für jeden Bindungsantikörper wird eine Testplatte benutzt. Jede Vertiefung der Testplatten wird mit 50 ul einer Lösung (a) beschickt, eine Stunde bei 37° C inkubiert und einmal mit physiologischem Phosphatpuffer PBS-Tween 20 gewaschen. Die Platten werden dann mit 200 ul pro Vertiefung der Waschlösung während 1 Stunde bei 37° C inkubiert und hierauf einmal wie zuvor gewaschen.

## Auftragen der Standardantigene

Nach dem Auftragen der Bindungsantikörper wird pro Testplatte ein Standardantigen in zwei 11er-Reihen (Verdünnungsreihen) wie folgt aufgetragen: In die erste Vertiefung werden 50 ul einer Lösung (c), entsprechend 1000 ng/ml, eingetragen. Die folgenden 10 Vertiefungen werden mit je 50 ul PBS-Tween 20 (siehe oben) beschickt. Zur Herstellung einer 1:2 Verdünnungsreihe wird die zweite Vertiefung mit 50 ul der Lösung (c) beschickt, durchgemischt, daraus 50 ul in die dritte Vertiefung übertragen und der Verdünnungsvorgang bis zur letzten Vertiefung wiederholt. Der letzten Vertiefung werden ebenfalls 50 ul entnommen und verworfen. In jeder Vertiefung befinden sich dann je 50 ul der Stardantigenlösung in den absteigenden Konzentrationen 1000, 500, 250, 125, 62.5, 31.25, 15.6, 7.8, 3.9, 1.9, und 0.98 ng/ml. Zu dieser Verdünnungsreihe wird eine analoge zweite Reihe angelegt (siehe **FIG. 1**).

Pro Antigen wird eine Platte benutzt. Das rekombinante Antigen MRP 8 wird auf die Testplatte 1, MRP 14 auf die Testplatte 2 und der Antigenkomplex MRP 8 - MRP 14 auf die Testplatte 3 aufgebracht.

## Auftragen der Proben

Die Proben der Körperflüssigkeiten, in denen der Gehalt an natürlichen Antigenen MRP 8, MRP 14 und dem Komplex MRP 8-MRP 14 (L-1) bestimmt werden soll, werden entweder unverdünnt oder, je nach vermutetem Gehalt an Antigen, mit PBS-Tween 20 verdünnt. EDTA-Plasma kann ungefähr 1/10 bis 1/100, Synovialflüssigkeit etwa 1/100 bis 1/10,000, Speichel etwa 1/100 und Exhalat unverdünnt oder bis zu 1/5 verdünnt werden. Nach Beschickung mit 50 ul pro Vertiefung mit den Lösungen der Proben, werden die Platten 1 Stunde bei 37°C inkubiert und dreimal in PBS-Tween 20 gewaschen. Jede Probe wird auf alle drei Testplatten aufgetragen.

## Auftragen der biotinylierten Nachweisantikörper

Die Vertiefungen auf allen Platten werden jetzt mit je 50 ul der Lösung (b) der entsprechenden Naschweisantikörper beschickt und die Platten, nach Inkubation während einer Stunde bei 37°C, dreimal in PBS-Tween 20 gewaschen.

**Markierung mit Extravidin-Peroxidase**

Zur Markierung der gebildeten Antikörper-Antigen-Antikörper Komplexe wird jede Vertiefung mit 50 ul Extravidin-Peroxidase, verdünnt nach Vorschrift des Lieferanten mit PBS-Tween 20, beschickt und nach 30 Minuten Inkubation bei 37° C dreimal in PBS-Tween 20 gewaschen.

**Auftragen des Substrates für die Peroxidase**

Zehn mg ABTS [2,2'-Azino-bis(3-ethylbenzthiazolin-6-sulfonsäure)] werden in 25 ml 50 mM Natriumcitratpuffer pH 4 gelöst und der benötigte Anteil davon unmittelbar vor dem Gebrauch mit 10 ul Wasserstofsuperoxid pro Platte versetzt. Von dieser Lösung werden 200 u pro Vertiefung aufgetragen und 20 Minuten bei Raumtemperatur inkubiert.

**Auswertung**

Die Auswertung erfolgt durch Messung der Extinktionen bei 405 nm, z. B. mittels eines EIA-Readers. Die Mittelwerte der Extinktionen der beiden Reihen der Standardantigene werden gegen die Konzentrationen der Standardantigene zu Kurven aufgetragen, die als Eichkurven zur Bestimmung der Konzentrationen der entsprechenden natürlichen Antigene in den Proben dienen. Drei typische Eichkurven sind in **FIG. 2** abgebildet.

Die Konzentrationen der Antigene in den Proben sollten so gewählt werden, dass die sich ergebenden Extinktionen im Bereich des steilen Kurvenanstieges liegen. Der Gehalt der Proben an den natürlichen Antigenen kann dann mit guter Genauigkeit abgelesen, bzw. unter Berücksichtigung der Verdünnungen berechnet werden.

Mittels der **Tabelle 2** wird die Zuordnung getroffen, ob es sich um einen Normalfall, d. h. gesunden Patienten, oder einen Patienten mit akuter Entzündung, chronischer Entzündung mit akutem Schub oder chronischer Entzündung ohne akuten Schub handelt.

**Beispiel 4: Messungen in Synovialflüssigkeiten**

In Synovialflüssigkeiten von 59 Patienten mit unterschiedlichen rheumatologischen Krankheitssymptonen wurden die in der **Tabelle 4** aufgeführten Werte gemessen.

| Tabelle 4 | | | |
|---|---|---|---|
| Symptome | MRP 8 ng/ml | MRP 14 ng/ml | MRP 8/14 ng/ml |
| Traumatische Ergüsse | | | |
| nicht entzündlich | 0 | 0-50 | 1500-2000 |
| entzündlich | 0 | 0-30 | 30000-50000 |
| Arthrosen | | | |
| nicht entzündlich | 0 | 10-30 | 200-1000 |
| entzündlich | 0 | 70-2000 | 4000-900000 |
| Arthritis | 0 | 1000 | 45000-300000 |
| Psoriasis Arthritis (1 Patient) | 34 | 40 | 45000 |
| Chronische Polyarthritis | 20-1600 | 26-15000 | 18000-9000000 |

**Patentansprüche**

1. Eine diagnostische Testpackung zur Bestimmung und Unterscheidung von akuten und chronischen entzündlichen Zuständen, enthaltend

    a) als Standardantigene, die rekombinanten Peptide MRP 8, MRP 14 und den Komplex aus MRP 8 und MRP 14,
    b) als Bindungsantikörper, die monoklonalen Antikörper Mab 8-5C2, Mab S-36.48 und Mab 27E10,
    c) als Nachweisantikörper, von Mab S 13.67, Mab S 32.2 und Mab S 36.48 abgeleitete, zum Nachweis geeignet derivatisierte monoklonale Antikörper und gegebenfalls
    d) ein Reagenz zum Sichtbarmachen der Nachweisantikörper.

2. Eine diagnostische Testpackung nach Anspruch 1, dadurch gekennzeichnet, dass die Nachweisantikörper Fluoreszenzderivate sind, wie solche hergestellt mit Fluoresceinisothiocyanat (FITC) oder Phycoerythrin (PE).

3. Eine diagnostische Testpackung nach Anspruch 1, dadurch gekennzeichnet, dass die Nachweisantikörper durch ein Enzym, wie Meerrettichperoxidase, Phosphatase, Glucosidase oder Galactosidase, derivatisiert sind.

4. Eine diagnostische Testpackung nach Anspruch 1, dadurch gekennzeichnet, dass die Nachweisantikörper durch Biotin derivatisiert sind.

5. Eine diagnostische Testpackung nach Anspruch 1, dadurch gekennzeichnet, dass sie die Bestandteile a), b) und c) in lyophilisierter Form umfasst.

6. Eine diagnostische Testpackung nach Anspruch 1, dadurch gekennzeichnet, dass sie die Fluoreszenzderivate der Nachweisantikörper in flüssiger Form umfasst.

7. Eine diagnostische Testpackung nach Anspruch 1, dadurch gekennzeichnet, dass sie 1-20 Vials mit standardisierten Mengen MRP 8, MRP 14 und eines Komplexes aus MRP 8 und MRP 14 im Verhältnis 2:1 umfasst.

8. Eine diagnostische Testpackung nach Anspruch 1, dadurch gekennzeichnet, dass sie
    2 Vials enthaltend je 250 ng rekombinantes MRP 8,
    2 Vials enthaltend je 250 ng rekombinantes MRP 14,
    2 Vials enthaltend je 250 ng einer Mischung aus MRP 8 und MRP 14 im Verhältnis 2:1,
    1 Vial enthaltend 60 ug Bindungsantikörper Mab 8-5C2,
    1 Vial enthaltend 90 ug Bindungsantikörper Mab S 36.48,
    1 Vial enthaltend 60 ug Bindungsantikörper Mab 27E10,
    1 Vial enthaltend 15 ug biotinylierten Mab S 13.67,
    1 Vial enthaltend 15 ug biotinylierten Mab S 32.2, und
    1 Vial enthaltend 15 ug biotinylierten Mab S 36.48 umfasst.

9. Eine diagnostische Testpackung nach Anspruch 1, dadurch gekennzeichnet, dass sie
    10 Vials enthaltend je 250 ng rekombinantes MRP 8,
    10 Vials enthaltend je 250 ng rekombinantes MRP 14,
    10 Vials enthaltend je 250 ng einer Mischung aus MRP 8 und MRP 14 im Verhältnis 2:1,
    1 Vial enthaltend 220 ug Bindungsantikörper Mab 8-5C2,
    1 Vial enthaltend 330 ug Bindungsantikörper Mab S 36.48,
    1 Vial enthaltend 220 ug Bindungsantikörper Mab 27E10,
    1 Vial enthaltend 100 ug biotinylierten Mab S 13.67,
    1 Vial enthaltend 100 ug biotinylierten Mab S 32.2, und
    1 Vial enthaltend 50 ug biotinylierten Mab S 36.48 umfasst.

10. Eine diagnostische Testpackung nach Anspruch 1, dadurch gekennzeichnet, dass sie Extravidin-Peroxidase umfasst.

11. Eine diagnostische Testpackung nach Anspruch 1, dadurch gekennzeichnet, dass sie als als Reagenz zum Sichtbarmachen 2,2'-Azino-bis-(3ethylbenzthiazolin-6-sulfonsäure) umfasst.

12. Eine diagnostische Testpackung nach Anspruch 1, dadurch gekennzeichnet, dass sie eine Anweisung zur Benutzung umfasst.

13. Verfahren zur Herstellung einer diagnostischen Testpakkung gemäss Anspruch 1, dadurch gekennzeichnet, dass man sie auf konventionelle Weise herstellt.

14. Verwendung einer Kombination
    a) der rekombinanten Peptide MRP 8, MRP 14 und des Komplexes aus MRP 8 und MRP 14 als Standardantigene,
    b) der monoklonalen Antikörper Mab 8-5C2, Mab S-36.48 und Mab 27E10 als Bindungsantikörper und
    c) von Mab S 13.67, Mab S 32.2 und Mab S 36.48 abgeleiteter, zum Nachweis geeignet derivatisierter monoklonale Antikörper als Nachweisantikörper, zur Herstellung einer diagnostischen Testpackung zur Bestimmung und Unterscheidung von akuten und chronischen entzündlichen Zuständen.

15. Verwendung einer Kombination
    a) der rekombinanten Peptide MRP 8, MRP 14 und des Komplexes aus MRP 8 und MRP 14 als Standardantigene,
    b) der monoklonalen Antikörper Mab 8-5C2, Mab S-36.48 und Mab 27E10 als Bindungsantikörper und
    c) von Mab S 13.67, Mab S 32.2 und Mab S 36.48 abgeleiteter, zum Nachweis geeignet derivatisierter monoklonale Antikörper als Nachweisantikörper, zur Bestimmung und Unterscheidung von akuten und chronischen entzündlichen Zuständen.

16. Diagnostische Methode zur Bestimmung und Unterscheidung von akuten und chronischen entzündlichen Zuständen in Körperflüssigkeiten, dadurch gekennzeichnet, dass man mit Hilfe der rekombinanten Standardantigene MRP 8, MRP 14 bzw. der Mischung dieser beiden Antigene im Molverhältnis 2:1, den Bindungsantikörpern Mab 8-5C2, Mab S-36.48 bzw. Mab 27E10 und den zum Nachweis geeignet derivatisierten Mab S 13.67, Mab S 32.2 bzw. Mab S 36.48 für die drei Antigene Eichkurven erstellt, in einer geeigneten Körperflüssigkeit die darin vorhandene Menge von MRP8, MRP 14 und dem Heterokomplex von MRP 8 und MRP 14 mittels der monoklonalen Bindungsantikörper und derivatisierten monoklonalen Nachweisantikörper durch einen immunologischen Test bestimmt, und durch Vergleich mit der Standardkurve und einer Tabelle die Auswertung vornimmt.

|   | 1 | 2 | 3 | 4 | 5 | 6 | 7 | 8 | 9 | 10 | 11 | 12 |
|---|---|---|---|---|---|---|---|---|---|----|----|----|
| A |   | 0,98 | 1,9 | 3,9 | Standard | ( | ng / ml | ) | 125 | 250 | 500 | 1000 |
| B | B | 0,98 | 1,9 | 3,9 | 7,9 | 15,6 | 31,25 | 62,5 | 125 | 250 | 500 | 1000 |
| C | l | 1 |   |   |   |   |   |   |   |   |   |   |
| D | a |   |   |   |   |   |   |   |   |   |   |   |
| E | n |   |   | Samples |   |   |   |   |   |   |   |   |
| F | k |   |   |   |   |   |   |   |   |   |   |   |
| G |   |   |   |   |   |   |   |   |   |   |   |   |
| H |   |   |   |   |   |   |   |   |   |   |   |   | 66 |

FIG. 1

FIG. 2

Europäisches
Patentamt

**EUROPÄISCHER RECHERCHENBERICHT**

Nummer der Anmeldung

EP 93 81 0561

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.5) |
|---|---|---|---|
| X | EP-A-0 263 072 (CIBA-GEIGY AG)<br><br>* Seiten 1-5; Seiten 22-27. *<br>--- | 1-6,<br>13-16 | G01N33/68<br>G01N33/564 |
| A,D | EUROPEAN JOURNAL OF IMMUNOLOGY<br>Bd. 19, Nr. 6 , 1989<br>Seiten 1053 - 1061<br>N.HOGG ET AL. 'Monoclonal antibody 5.5 reacts with p8,14, a myeloid molecule associated with some vascular endothelium.'<br>* Seite 1053 - Seite 1054 *<br>--- | 1-4 | |
| A,D | CLINICAL AND EXPERIMENTAL IMMUNOLOGY<br>Bd. 72, Nr. 3 , Juni 1988<br>Seiten 510 - 515<br>G.ZWALDO ET AL. 'Two calcium-binding proteins associated with specific stages of myeloid differentiation are expresed by subsets of macrophages in inflammatory tissues.'<br>* das ganze Dokument *<br>--- | 1,3 | |
| | | | RECHERCHIERTE SACHGEBIETE (Int.Cl.5) |
| | | | G01N<br>C07K |
| A,D | EUROPEAN HEART JOURNAL<br>Bd. 11, Nr. 7 , Juli 1990<br>Seiten 619 - 627<br>B.MUES ET AL. 'Phenotyping of macrophages with monoclonal antibodies in endomyocardial biopsies as a new approach to diagnosis of myocarditis.'<br>* das ganze Dokument *<br>--- | 1-4 | |
| A | US-A-4 968 604 (S.M.BEATTY)<br><br>* Spalte 8 *<br>---<br><br>-/-- | 1-4,10,<br>11 | |

Der vorliegende Recherchenbericht wurde für alle Patentansprüche erstellt

| Recherchenort | Abschlußdatum der Recherche | Prüfer |
|---|---|---|
| DEN HAAG | 18. November 1993 | HITCHEN, C |

**Europäisches Patentamt**

# EUROPÄISCHER RECHERCHENBERICHT

Nummer der Anmeldung

EP 93 81 0561

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.5) |
|---|---|---|---|
| A,P | IMMUNOBIOLOGY<br>Bd. 186, Nr. 3-4 , November 1992<br>Seiten 304 - 314<br>J.ROTH ET AL. 'Complex Pattern of the Myelo-Monocytic Differentiation Antigens MRP8 and MRP14 during Chronic Airway Inflammation.'<br>* das ganze Dokument *<br>--- | 1,3-6,<br>10-13 | |
| A,P | ATEMWEGS- UND LUNGENKRANKHEITEN<br>Bd. 18, Nr. 9 , September 1992<br>Seiten 349 - 352<br>J.ROTH ET AL. 'Die kalziumbindenden Proteine MRP8 und MRP14: Zwei neue Marker zur Monozyten-differenzierung in Entzuendungen.'<br>* das ganze Dokument *<br>----- | 1,3,4,<br>10-13 | |
| | | | **RECHERCHIERTE SACHGEBIETE (Int.Cl.5)** |

Der vorliegende Recherchenbericht wurde für alle Patentansprüche erstellt

| Recherchenort | Abschlußdatum der Recherche | Prüfer |
|---|---|---|
| DEN HAAG | 18. November 1993 | HITCHEN, C |

EPO FORM 1503 03.82 (P04C03)